# EUROPEAN PATENT APPLICATION

(11) **EP 4 439 449 A1**
(43) Date of publication of application: **02.10.2024**
(21) Application number: 23164876.7
(22) Date of filing: 29.03.2023
(51) Int. Cl.: G06T 7/00

(54) **METASTATIC CHARACTERIZATION USING NEURAL NETWORK AND APPARENT DIFFUSION COEFFICIENT MAP**

(71) Applicant: Siemens Healthineers AG, 91301 Forchheim (DE)
(72) Inventor: Datar, Manasi, 99084 Erfurt (DE)
(74) Representative: Siemens Healthineers Patent Attorneys

(57) **Abstract**

Various examples of the disclosure pertain to the characterization of one or more metastases using a neural network, as well as an apparent diffusion coefficient, ADC, map as obtained from diffusion-weighted magnetic resonance imaging, DWI MRI. A convolutional neural network can be employed. Training processes and inference processes are disclosed.

## Description

### TECHNICAL FIELD

Various examples of the disclosure generally pertain to metastatic characterization using a neural network. Various examples of the disclosure specifically relate to using an apparent diffusion coefficient map as obtained from a diffusion weighted imaging measurement using magnetic resonance imaging when predicting the metastatic characterization using the neural network.

### TERMINOLOGY

Independent of the grammatical term usage, individuals with male, female or other gender identities are included within the term.

### BACKGROUND

Magnetic resonance imaging (MRI) is a prime modality for cancer staging to determine the optimal therapy and/or surgical treatment option. The cancer staging is typically based on markers (characterization coefficients) such as the extent of the primary tumor (T-stage), spread of the cancer (metastasis) to regional lymph nodes (N-stage), and the metastasis of cancer to distant sites (M-stage). The N-stage coefficient and M-stage coefficient can take, e.g., scalar values of 0, 1 or 3.

Detection of metastatic conditions including lymph node metastasis and presence of distant metastatic lesions are helpful for treatment planning and prognosis prediction. For example, detection and visualization of changes in metastatic lesions (M-stage) is an important criterion in (i) determination of effective treatment options for treating patients with advanced cancers, (ii) follow up of patients whose primary tumors have been treated, and (iii) monitoring patient response to ongoing therapy/treatment. See M. Menezes , S. Das, I. Minn , L. Emdad , X. Wang , D. Sarkar , M. Pomper and P. Fisher, "Detecting Tumor Metastases: The Road to Therapy Starts Here," Adv Cancer Res, 2016.

Based on the metastasis characterization, about 50% of advanced cancer patients receive neoadjuvant therapy to treat potential lymph node metastases. The disadvantage is that the radiation exposure may affect the accuracy of post-treatment follow-up staging despite the established fact that disease free survival correlates with lymphatic invasion.

N-stage determination with high accuracy remains a challenge. Various criteria (size, shape, type of boundary, etc.) are widely discussed without further consensus on accurate N-stage diagnosis. See, e.g., Z. Zhuang, Y. Zhang, M. Wei, X. Yang and Z. Wang, "Magnetic Resonance Imaging Evaluation of the Accuracy of Various Lymph Node Staging Criteria in Rectal Cancer: A Systematic Review and MetaAnalysis," Frontiers in Oncology, 2021. Although accuracy of metastasis detection is better with MRI compared to other imaging modalities, challenges remain due to (i) the 'flare' phenomenon post therapy, (ii) presence of lesions in close proximity, and (iii) high variability in lesion characteristics due to the advanced disease state. See M. Menezes , S. Das, I. Minn , L. Emdad , X. Wang , D. Sarkar , M. Pomper and P. Fisher, "Detecting Tumor Metastases: The Road to Therapy Starts Here," Adv Cancer Res, 2016.

To mitigate such issues, advanced algorithms are used to provide metastatic characterization.

For example, artificial intelligence techniques based on deep neural networks (or simply neural networks; NNs) have been applied to metastasis detection, both lymph node metastasis and metastatic lesion detection, with some success. See Q. Zheng, L. Yang, B. Zeng, J. Li, K. Guo, Y. Liang and G. Liao, "Artificial intelligence performance in detecting tumor metastasis from medical radiology imaging: A systematic review and meta-analysis," EClinicalMedicine, 2021.

Further, beyond such developments in the algorithmics to provide metastatic characterization, also imaging protocols have been developed to provide better results. For example, contrast-enhanced (CE) MRI with diffusion weighted imaging (DWI) has been increasingly used in recent clinical practice for detection and characterization of metastasis. Lesion enhancement patterns from CE-MRI are useful in detection, whereas correlation with radiomics features and/or biomarkers such as apparent diffusion coefficient (ADC) map have been increasingly useful for characterization of metastases and for treatment response prediction. See Q. Hu, G. Wang, X. Song, J. Wan, M. Li, F. Zhang, Q. Chen, X. Cao, S. Li and Y. Wang, "Machine Learning Based on MRI DWI Radiomics Features for Prognostic Prediction in Nasopharyngeal Carcinoma," Cancers, 2022.

Such techniques face certain restrictions and drawbacks. For instance, candidate regions in which metastasis is to be detected by a NN are annotated manually. This can be inaccurate. The detection and characterization of metastasis remains a two-step process in current clinical practice.

### SUMMARY

Accordingly, a need exists for advanced techniques of predicting metastatic characterization based on deep neural networks.

This need is met by the features of the independent claims. The features of the dependent claims define embodiments.

A computer-implemented method includes obtaining and ADC map. The apparent diffusion coefficient map is for a region of interest of the patient. The ADC map is determined based on a DWI MRI measurement. The computer-implemented method also includes performing a training process of a deep convolutional neural network. The deep convolutional neural network is trained to make predictions of a metastatic characterization based on MRI images of the region of interest. The deep convolutional neural network includes multiple layers. The deep convolutional neural network also includes one or more attention gates. The one or more attention gates prioritize amongst activations of the respective layer of the deep convolutional network. The prioritizing is based on respective self-attention maps. The self-attention maps capture a spatial context of the respective layer. The training process is based on first ground-truth data that is indicative of the metastatic characterization. The training process is further based one second ground-truth data to train the one or more attention gates. The second round-truth data is based on the ADC map.

Such trained deep convolutional neural network can be employed in one or more of the following methods, for inference.

A computer-implemented method includes obtaining an ADC map for a region of interest of a patient. The ADC map is determined based on a DWI MRI measurement. The method also includes obtaining an MRI image. The MRI image is determined based on the DWI MRI measurement or based on another MRI measurement. The method further includes making a prediction of a metastatic characterization. The prediction is made using a deep convolutional neural network. The deep convolutional neural network includes multiple layers and one or more attention gates. Each of the one or more attention gates prioritizes amongst activations of a respective layer of the deep convolutional neural network. Such a prioritization is based on a respective self-attention map that captures a spatial context of the respective layer. The method also includes determining a reliability of the prediction based on a comparison between each self-attention map of the one or more attention gates and a response and a representation of the ADC map.

A computer-implemented method includes obtaining an ADC map for a region of interest of a patient. The ADC map is determined based on a DWI MRI measurement. The method also includes obtaining an MRI image. The MRI image is determined based on the DWI MRI measurement are based on another MRI measurement. The method also includes making a prediction of a metastatic characterization using a deep convolutional neural network. One or more layers of the deep convolutional neural network obtain, as a respective input, respective spatial context information. The respective spatial context information is determined based on the ADC map.

Program code can be executed by at least one processor. Execution of the program code causes the processor to perform such methods as disclosed above.

A processing device includes a processor and a memory. The processor is configured to load program code from the memory and to execute the program code. Execution of the program code causes the processor to perform such methods as disclosed above.

It is to be understood that the features mentioned above and those yet to be explained below may be used not only in the respective combinations indicated, but also in other combinations or in isolation without departing from the scope of the disclosure.

### BRIEF DESCRIPTION OF THE DRAWINGS

FIG. 1 schematically illustrates a processing device according to various examples.
FIG. 2 is a flowchart of a method according to various examples.
FIG. 3 schematically illustrates a neural network for metastatic characterization according to various examples.
FIG. 4 is a flowchart of a method according to various examples.

### DETAILED DESCRIPTION

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the disclosure will be described in detail with reference to the accompanying drawings. It is to be understood that the following description of embodiments is not to be taken in a limiting sense. The scope of the disclosure is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

In current clinical practice, imaging studies for cancer staging include CE-MRI, along with DWI imaging and ADC map calculation. While automated algorithms to detect lymph nodes and lesions may be used, the crucial step of metastatic characterization, e.g., N-stage and M-stage metastatic characterization of one or more metastases, is done manually in current clinical processes. ADC maps are used retrospectively by the practitioner to inform the characterization and staging.

Various techniques disclosed herein employ ADC maps as a spatial prior for end-to-end, automated detection and characterization of metastases based on MRI images, e.g., obtained from CE-MRI measurements.

According to various examples, a NN is employed to make the prediction of the metastatic characterization, e.g., N-stage and/or M-stage. A convolutional NN can be employed. A U-net architecture can be employed. See Siddique, Nahian, et al. "U-net and its variants for medical image segmentation: A review of theory and applications" Ieee Access (2021): 82031-82057.

According to various examples, the NN includes one or more attention gates. As a general rule, an attention gate prioritizes amongst activations of the respective hidden layer of the NN based on a self-attention map that captures a spatial context of the respective hidden layer. The self-attention map is a single channel image capturing the spatial distribution of the attention coefficient of the respective layer of the NN that encodes the relative importance of each image location. The attention gate can include one or more convolution layers, each followed by a rectifier linear unit (ReLu) activation. This can be followed by a sigmoid activation and next multiplied with the input feature map at each layer to generate the attention map ***A_{I}*.**

An example implementation of attention gates is disclosed in: O. Oktay, J. Schlemper, L. Folgoc, M. Lee, M. Heinrich, K. Misawa, K. Mori, S. McDonagh, N.Hammerla, B. Kainz, B. Glocker and D. Rueckert, "Oktay, O., Schlemper, J., Folgoc, L.L., Lee, M.J., Heinrich, M.P., Misawa, K., Mori, K., McDonagh, S.G., Hammerla, N.Y., Kainz, B., Glocker, B., & Rueckert, D. (2018). Attention U-Net: Learning Where to Look for the Pancreas," ArXiv, 2018.

There are multiple options available for considering the ADC map in the context of the metastatic characterization facilitated by the NN.

In a first option, an ADC map is used for explicit supervision of attention map learning. This means that the ADC is used in a training process that sets parameters of the self-attention map. The corresponding attention loss can be formulated as a standard or masked regression loss and added to the final loss function of the network. In general, the masked regression loss is computed only at relevant points in the image. In the context of the disclosed techniques, the ground-truth ADC map may be binarized using a threshold to create a mask and the loss calculations may be restricted to points where the mask value is 1.The ADC map can be resampled to match the dimensions of the attention map at each hidden layer and therefore provide a (pseudo) ground truth ***A_{G}*** for spatial importance of each location in the image. A loss can be formulated to measure the difference between ***A_{I}*** and ***A_{G}**.* During the training process, this loss can drive the learned attention maps as close to the ADC maps as possible. Accordingly, the training process for the NN is not only based on a first ground-truth data that is indicative of the metastatic characterization, e.g., N-stage or M-stage (e.g., obtained from manual annotation by a domain expert). Rather, the training process is also based on the second ground-truth data to train the one or more attention gates. This second ground-truth data is based on the ADC map. For this, the ADC map is re-sampled for each of the one or more attention gates, to a respective spatial grid that is associated with the respective attention gate. For each of the one or more attention gates, a respective portion of the second ground-truth data is accordingly determined based on the ADC map.

In a second option, - once the training process is completed, during inference - it is possible to determine a reliability of the prediction made by the NN based on a comparison between each self-attention map of the one or more attention gates and the representation of the ADC map. In other words, it can be determined whether the self-attention map or self-attention maps of one or more attention gates of the NN appropriately match the respective resampled representation of the ADC map. If there is a significant deviation, then it can be assumed that the output provided by the NN (and along with it the metastatic characterization) is unreliable. This approach works particularly well for a NN that has been subject to a training process as explained above, i.e., subject to a training process that is based on a ground-truth data that is based on the ADC map. Here, any deviation between the actual ADC map measured during inference and the self-attention maps of the one or more attention gates indicates operation of the NN out of distribution, i.e., outside of the particular input space for which it has been trained.

In a third option, the ADC map is used as a spatial prior during inference. The ADC map obtained from a respective DWI measurement can be reshaped or passed through a parallel set of convolutional layers to obtain ADC feature maps (encoding the ADC map in a latent space). The ADC feature maps are then concatenated with the input feature maps at one or more layers of the NN. In such approach, the ADC feature maps act as spatial priors and provide the NN with additional information about the probability of each location being relevant in the detection and characterization of metastasis. Accordingly, in other words, one or more hidden layers of the deep convolutional NN obtain, as a respective input, respective spatial context information that is determined based on the ADC map. This spatial context information can be determined for each of the one or more hidden layers using one or more convolutional layers to encode the ADC map.

Above, three options have been disclosed that enable to employ an ADC map as additional information in the framework of metastatic characterization based on an NN, e.g., a CNN. These three options can be combined with each other or used in isolation.

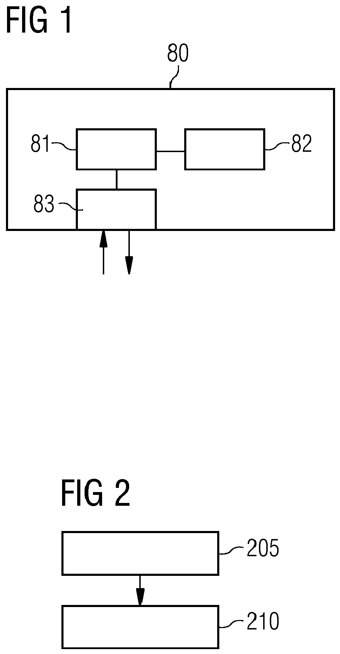

FIG. 1 schematically illustrates a processing device 80 according to various examples. The processing device 80 includes a processor 81 and a memory 82. The processor 81 can load program code that is stored in the memory 82 and can execute the program code. The processor 81, upon executing the program code, performs techniques as disclosed herein, e.g.: performing a training process of a NN so that the deep convolutional NN is trained to predict a metastatic characterization based on MRI images; obtaining MRI images from an MRI apparatus via a communication interface 83; outputting the metastatic characterization via the interface 83; executing inference to predict the metastatic characterization using the pre-trained NN; using ADC maps when making the metastatic characterization,; etc.

FIG. 2 schematically illustrates a flowchart of a method according to various examples. The method of FIG. 2 pertains to the different stages of metastatic characterization using a NN according to various examples.

At box 205, the deep convolutional NN is trained using a respective training process. The training process is based on one or more losses.

A first loss considers a difference between an output of the NN and first ground-truth data. The first ground-truth data is indicative of the metastatic characterization to be predicted by the NN. For instance, a user may manually annotate the N-stage or the M-stage as the first ground-truth data.

According to some examples, a further, second loss is considered. This further loss is based on second ground-truth data. The second ground-truth data is used to train the one or more attention gates. In particular, weights of the one or more attention gates are set based on the second loss. The second loss can compare the self-attention map used by each attention gate with a respective portion of the second ground-truth data that is based on the ADC map. The ADC map is re-sampled one or more times to a spatial grid that is associated with each attention gate to determine a respective portion of the second ground-truth data for each of the one or more attention gates.

In other words, each attention gate is operating based on a self-attention map that has a certain spatial grid and, to be able to compare the self-attention map with the second ground-truth data the ADC map is resampled to this spatial grid of the self-attention map. The loss can be a masked regression loss.

By such techniques, weights of the one or more attention gates - e.g., defining a kernel for a convolution, e.g., with the feature map of the respective hidden layer associated with the one or more attention gates - are trained to resample the ADC map (or more specifically the respective representation of the ADC map at the associated spatial grid).

In box 210 (inference), the trained deep convolutional NN is then used to make a prediction of a metastatic characterization based on one more MRI images. For instance, N-stage or M-stage scoring values (e.g., "0", "1" or "3") can be determined.

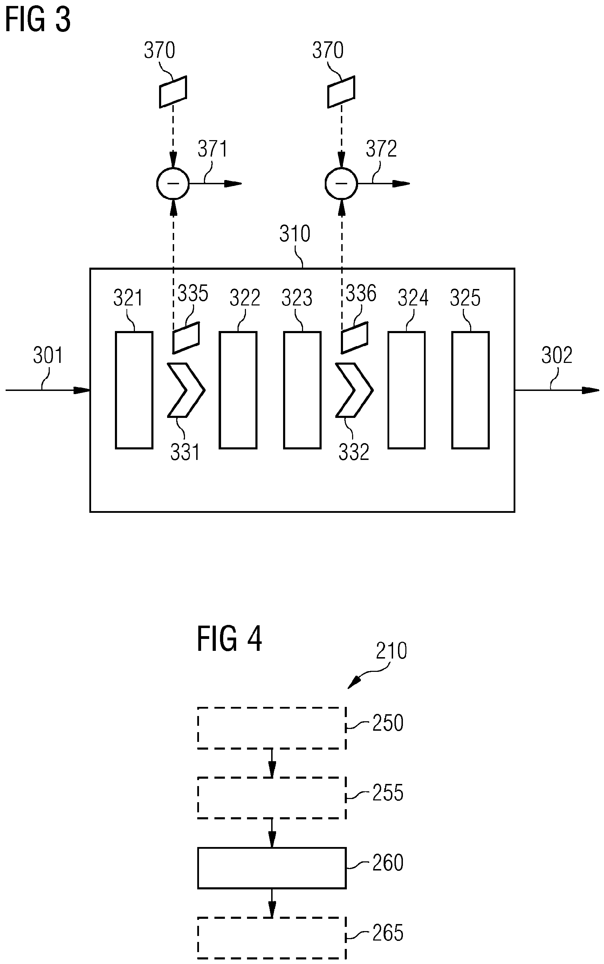

FIG. 3 schematically illustrates a NN 310 that can be used according to various examples to make a prediction of a metastatic characterization (the design of the NN 310 is only an illustration; e.g., the NN 310 may include more or fewer layers and/or more or fewer attention gates). For instance, the NN 310 can be used as part of box 205 and/or of box 210 in FIG. 2.

For example, the NN 310 can implement a U-net architecture. One or more of the layers 321-325 can employ convolutions and/or max-pooling operations and/or nonlinear activations. Kernels of such convolutional layers can be trained in the training process (cf. FIG. 2: box 205).

The NN 310 obtains, as an input, one or more MRI images 301. The one or more MRI images 301 are obtained in an MRI measurement. The MRI measurement can be part of an MRI exam that includes multiple MRI measurements. The MRI images can be anatomical MRI images or CE-MRI images. The MRI images can be diffusion weighted images.

The NN 310 provides, as an output, the metastatic characterization 302, e.g., the N-stage or M-stage. This corresponds to a classification task.

The NN 310 includes multiple layers 321-325. At least some of these layers 321-325 can be convolutional layers.

The NN 310 also includes multiple attention gates 331-332. These attention gates 331, 332 prioritize amongst activations of a respective layer of the NN based on respective gate-specific self-attention maps 335, 336 that capture a respective spatial context of the respective layer. For instance, the layer 323 provides an activation map as its output and the different activations of this activation map of the layer 323 are then suppressed or amplified by the attention gate 332, before being input to a subsequent layer 324. The attention gates 331, 332, accordingly, act as filters that provide spatially dependent filtering. This suppression or amplification implements a local prioritization. The suppression or amplification is in accordance with attention weights defined by the self-attention map 336 associated with the attention gate 332. The self-attention map 336 can be determined based on the activation of one or more of the preceding layers 321-323. The self-attention map 336 can be determined by convolution with a trained kernel. Respective training techniques to obtain the trained kernel have been discussed above in connection with box 205.

Also illustrated in FIG. 3 is a comparison of each of the self-attention maps 335, 336 with a respective representation of the ADC map 370, e.g., obtained in the same MRI measurement or another MRI measurement of the same MRI examples that also is used to obtain the MRI image 301. It is possible to compare the representation of the ADC map 370 with each of the self-attention maps 335, 336 to obtain respective indicators 371, 372 indicative of a deviation between the self-attention maps 335, 336 and the representations of the ADC map 370. A pixel-by-pixel comparison can be made. Based on these indicators 371, 372 it is then possible to determine a reliability of the prediction of the NN 310, i.e., a reliability of the metastatic characterization 302. In particular, where a significant deviation is detected, a low reliability can be assumed.

FIG. 4 is a flowchart of a method according to various examples. The method of FIG. 4 corresponds to inference using a pre-trained NN such as the NN 310 of FIG. 3. FIG. 4 pertains to making a prediction of a metastatic characterization. FIG. 4 can implement box 210 of the method of FIG. 2.

The method of FIG. 4 can be executed by a processor upon loading program code from a memory and upon executing the program code. For instance, the method of FIG. 4 can be executed by the processor 81 of the processing device 80 upon loading program code that is stored in the memory 82 and upon executing the program code.

At box 250, it is optionally possible to acquire a diffusion weighted imaging MRI measurement data. Conventional protocols known in the art can be employed. Based on the acquired diffusion weighted imaging MRI data, an ADC map is determined. In other examples, the ADC map is pre-determined. The ADC map can then be loaded from a memory or database.

Optionally, at box 255, one or more MRI images are acquired. Further MRI measurements can be executed, e.g., as part of the same MRI exam including the diffusion weighted imaging MRI data acquisition at box 250. In other examples, diffusion weighted MRI images already acquired at box 250 can be used for making the prediction of the metastatic characterization. Also, pre-acquired MRI images can be used that are loaded from a memory or database.

At box 260, a prediction of the metastatic characterization is made using a NN.

It is possible that this prediction is based on an ADC map. The ADC map is determined based on the DWI measurement of box 250. In particular, it is possible that a spatial context information is determined based on the ADC map, e.g., by encoding the ADC map using one or more convolutional layers. Then, the spatial context information can be provided to one or more layers of the deep convolutional NN as context information, e.g., by concatenation with further input data.

Optionally, at box 265, a reliability of the prediction made by the NN is determined. This can be based on a comparison between one or more self-attention maps calculated in one or more attention gates of the NN and respective representations of the ADC map; respective aspects have been discussed above in connection with indicators 371, 372 in FIG. 3.

Summarizing, techniques have been disclosed that enable to take into consideration in ADC map during a training process and/or during inference of a NN that is used to make a prediction of a metastatic characterization. By using the ADC map, more reliable and fully automated predictions can be provided. The training process can be simplified and made more accurate. The accuracy of the prediction can be judged. The current clinical procedure of detecting lymph nodes and lesions manually can be simplified.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

## Claims

1. A computer-implemented method, comprising:
- obtaining an apparent diffusion coefficient, ADC, map (370) for a region of interest of a patient, the ADC map (370) being determined based on a diffusion-weighted magnetic resonance imaging, MRI, measurement,
- obtaining an MRI image (301) determined based on the diffusion-weighted MRI measurement or another MRI measurement, and
- making a prediction of a metastatic characterization using a deep convolutional neural network (310), one or more layers (321, 322, 323, 324, 325) of the deep convolutional neural network (310) obtaining, as a respective input, respective spatial context information determined based on the ADC map (370).

2. The computer-implemented method of claim 1, further comprising:
- for each of the one or more layers (321, 322, 323, 324, 325): determining the respective spatial context information by encoding the ADC map (370) using one or more convolutional layers.

3. A computer-implemented method, comprising:
- obtaining an apparent diffusion coefficient, ADC, map (370) for a region of interest of a patient, the ADC map (370) being determined based on a diffusion-weighted magnetic resonance imaging, MRI, measurement,
- obtaining an MRI image (301) determined based on the diffusion-weighted MRI measurement or another MRI measurement,
- making a prediction of a metastatic characterization using a deep convolutional neural network (310), the deep convolutional neural network (310) comprising multiple layers and one or more attention gates (331, 332) each prioritizing amongst activations of a respective layer (321, 322, 323, 324, 325) of the deep convolutional neural network (310) based on a respective self-attention map (335, 336) that captures a spatial context of the respective layer (321, 322, 323, 324, 325), and
- determining a reliability of the prediction based on a comparison between each self-attention map of the one or more attention gates (331, 332) and a representation of the ADC map (370).

4. A computer-implemented method, comprising:
- obtaining an apparent diffusion coefficient, ADC, map (370) for a region of interest of a patient, the ADC map being determined based on a diffusion-weighted magnetic resonance imaging, MRI, measurement, and
- performing (205) a training process of a deep convolutional neural network (310) to make predictions of a metastatic characterization based on MRI images (301) of the region of interest, the deep convolutional neural network (310) comprising multiple layers (321, 322, 323, 324, 325) and one or more attention gates (331, 332) prioritizing amongst activations of the respective layer (321, 322, 323, 324, 325) of the deep convolutional neural network (310) based on respective self-attention maps (335, 336) that capture a spatial context of the respective layer (321, 322, 323, 324, 325),
wherein the training process is based on first ground-truth data indicative of the metastatic characterization,
wherein the training process is further based on second ground-truth data to train the one or more attention gates (331, 332), the second ground-truth data being based on the ADC map (370).

5. The computer-implemented method of claim 4, further comprising:
- for each of the one or more attention gates (331, 332): re-sampling the ADC map (370) to a spatial grid associated with the respective one of the one or more attention gates (331, 332) to determine a respective portion of the second ground-truth data.

6. The computer-implemented method of claim 4 or 5,
wherein a loss of the training process associated with the second ground-truth data is a masked regression loss.

7. A computer program comprising program code executable by at least one processor, execution of the program code causing the processor (81) to perform the method of any one of claims 1 to 6.

8. A computer-readable medium comprising instructions which, when executed by a computer, cause the computer to carry out the method of any one of claims 1 to 6.

9. A processing device comprising a processor (81) and a memory (82), the processor being configured to load program code from the memory and to execute the program code, execution of the program code causing the processor (81) to perform the method of any one of claims 1 to 6.
